# EUROPEAN PATENT SPECIFICATION

(11) **EP 2 359 206 B1**
(45) Date of publication and mention of the grant of the patent: **31.08.2016**
(21) Application number: 09825145.7
(22) Date of filing: 30.04.2009
(51) Int. Cl.: G05D 7/00, A61L 2/18, B26D 1/143, B26D 1/16, A23B 4/18, B26D 1/00

(54) **PATHOGEN REDUCTION SYSTEM FOR THE PREPARATION OF SLICED FOOD PRODUCTS**
PATHOGENREDUKTIONSSYSTEM ZUR HERSTELLUNG VON IN SCHEIBEN GESCHNITTENEN NAHRUNGSMITTELPRODUKTEN
SYSTÈME DE RÉDUCTION DES PATHOGÈNES POUR LA PRÉPARATION DE PRODUITS ALIMENTAIRES EN TRANCHES

(30) Priority: 05.11.2008 US 111566 P
(43) Date of publication of application: 24.08.2011
(73) Proprietor: Spraying Systems Co., Wheaton, IL 60187-7901 (US)
(72) Inventor: RAMABADRAN, Arun, Naperville IL 60565 (US)
(74) Representative: Rüger, Barthelt & Abel
(86) International application number: PCT/US2009/042291
(87) International publication number: WO 2010/053595

(56) References cited:
- US-A- 3 927 588
- US-A- 5 802 959
- US-A1- 2002 192 340
- US-A1- 2004 018 284
- US-A1- 2004 101 604
- US-B1- 6 645 429

## Description

### CROSS-REFERENCE TO RELATED APPLICATION

This patent application claims the benefit of U.S. Provisional Patent Application No. 61/111,566 filed November 5, 2008.

### BACKGROUND OF THE INVENTION

When sliced meat products are stored, e.g., during transportation, on the store shelf prior to sale, or in the consumer's home, bacteria present in the product tend to multiply over time, eventually causing an unsafe condition for the consumer. In particular, certain bacterial and other pathogenic contaminations can be injurious or even deadly to bumans. For example, various strains of E coli. and Listeria bacteria have been know, when food-borne, to cause outbreaks of especially serious illnesses, especially in very young and very old consumers.

The predominant technique for controlling bacterial contamination in modem meat processing plants has been to disinfect the equipment that comes into contact with the food product, while maintaining high sanitation standards for production personnel, so as to minimize the transfer of bacteria. However, this technique docs not necessarily eliminate bacteria already present on the food product, and does not treat contamination that may occur between equipment cleaning cycles.

One previously proposed solution for inhibiting the proliferation of pathogens in packaged foods is described in US 2004/0101604 A1. Therein, a method is described in which two spray nozzles injecting a solution inhibiting pathogens arc arranged to treat sliced food. One of the spray nozzles is mounted above a belt carrying food slices opposite the cut off slice surface and is arranged to deposit the solution onto the slice surface of the food. The second spray nozzle is mounted above the level of the belt and to the side of the end face of the uncut food such that the solution deposited thereby is deposited onto the side of the uncut food and onto the back of the slice. Both nozzles are supplied with solution from the same source.

In US 5 802 959 A, a system for slicing potatoes is described, which includes depositing the potato slices in a bath to remove excess starch therefrom. US 6 645 429 B1 describes a sterilization system that involves fumigating a closed packaging room with a sterilizing agent that can be used to sanitize food packaging and the related packaging equipment. US 2004/0018284 A1 describes a method for controlling microbial contamination of a vacuum-sealed food product in which a surface water content of the food is adjusted before deposition of a microbial controlling solution.

### OBJECTS AND SUMMARY OF THE INVENTION

It is an object of the present invention to provide a food processing system and apparatus including a spraying apparatus with multiple spray heads throughout the system for controlling pathogenic contamination, eliminating a potential source of biological hazard for consumers.

Another object is to provide a food product processing system and apparatus as characterized above which can be operated on a substantially uninterrupted basis, without the need to frequently shut down the system for equipment cleaning.

A further object is to provide a processing system and apparatus of the foregoing type in which unintended disruption or malfunction of the spray components can be detected and an alarm provided to the operator of the system.

Other objects and advantages of the invention will become apparent upon reading the following detailed description and upon reference to the drawings, of which:

### BRIEF DESCRIPTION OF THE SEVERAL VIEWS OF THE DRAWINGS

Figure 1 is a schematic overview diagram of a generic meat slicing process within which the present invention may be implemented;
Figure 2 is a perspective view of a generic cutting operation, showing the surfaces exposed during the operation;
Figure 3 is a schematic diagram of a meat slicing system according to an embodiment of the present invention;
Figure 4 is a system schematic corresponding to a system within an embodiment of the present invention, including elements for user input and output, spray control, packaging control, and alarm control;
Figure 5 is a flow chart illustrating a process for initializing a meat processing system in an embodiment of the present invention; and
Figure 6 is a flow chart illustrating a process for meat processing in an embodiment of the present invention.

### DETAILED DESCRIPTION OF THE INVENTION

Bacterial contamination within a meat processing plant is a constant problem that must be addressed for the safety of the consumer. It is impossible to eliminate all bacteria in any processing plant, but it is possible to minimize contamination and control the growth of bacteria that remain. The extent of bacterial contamination in any given setting is typically referred to as "bacterial load." While the danger posed by a specific bacterial load is largely dependent on the type of bacteria in question, it is generally desirable to minimize bacterial load in a food processing plant or facility.

In today's food processing plants, many steps are taken in an attempt to minimize bacterial load in the finished food product. For example stringent standards for worker hygiene are enforced, and production equipment and areas are frequently cleaned and sterilized. Nonetheless, it is still all too common to have bacteria transferred to the product during processing prior to packaging. One particular piece of equipment in which bacterial load is difficult to control is within a deli meat slicer. Such devices have many surfaces and niches within which bacteria may be harbored. Such areas include grippers, inlet conveyors, product transfer conveyors, blades, and outlet conveyors. Moreover, the surface area of exposed meat provides another host for bacterial outgrowth in this application.

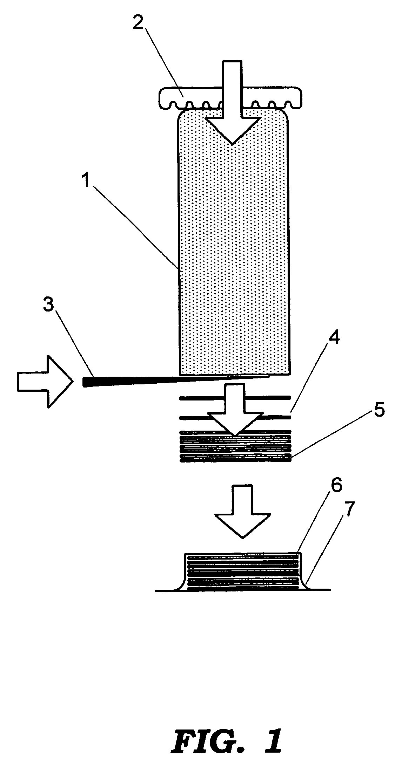

Referring more specifically to the figures, FIG. 1 shows a schematic overview diagram of a generic meat slicing process within which the present invention may be implemented. In general, a log 1 of food material such as meat, cheese, etc., is placed within the device in contact with a support 2. The log 1 typically rests on a table, conveyor, or platform, not shown. The function of the support 2 is to advance the log 1 into a slicer blade 3, shown schematically. The slicer blade 3 may be an oscillating straight blade, a rotating curved blade, or any other suitable blade, depending upon the cutting requirements imposed by the log 1 material and the speed of processing, as well as other factors potentially.

As the log 1 is advanced by the support 2 into the slicer blade 3, slices 4 of the log 1 are removed and accumulated, e.g., in a stack 5. When the stack 5 reaches a certain size or quantity, the finished stack 6 is moved away from the log 1 and is packaged within a package 7. The packaging may take any one of various known forms, but one often-used packaging type is the vacuum pack. This type of packaging generally employs a flexible envelope 7, alone or in conjunction with a backing piece. The finished stack 6 is inserted in the envelope 7, after which the envelope 7 is evacuated to a suitable vacuum level and scaled. The envelope 7 may also be backfilled with an inert gas if desired after evacuation.

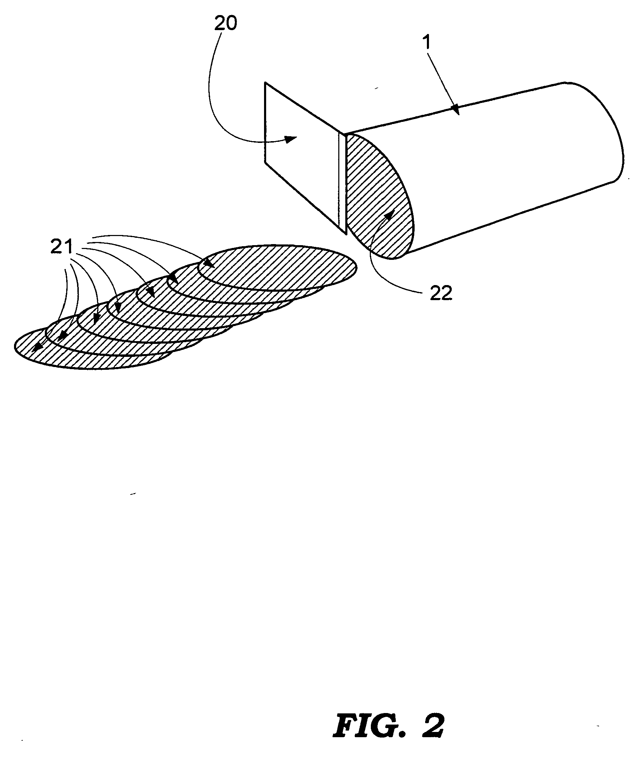

Having reviewed the basic slicing and packaging operation as it exists, the potential sources of contamination will be discussed with reference to FIG. 2. As can be seen, FIG. 2 illustrates a perspective view of a generic cutting operation, showing the surfaces exposed during the operation. As the slicer blade 3 removes each slice 4 from the log 1, a number of surfaces arc exposed for contamination or cross-contamination. In particular, each slice 4 presents a front surface 21, and a back surface (not shown). In addition, the exposed face 22 of the log 1 can receive bacteria from the blade 20, or can pass bacteria to the blade 20 to be deposited onto a subsequent slice. Thus, whereas the uncut log 1 exposes only a single outer surface, the sliced log 1 and slicer parts significantly multiply the number of surfaces on which contamination must be controlled.

In an embodiment of the invention, an antimicrobial fluid is applied, in a specifically controlled sequence, onto the various exposed faces of the processed food product during processing. From the teachings herein, it will be appreciated that it is desirable to apply a certain amount of the antimicrobial fluid, and to avoid applying substantially more or substantially less than that predetermined amount. In particular, the use of too little antimicrobial fluid raises the risk that the bacterial load may not be appropriately controlled, whereas the use of too much antimicrobial fluid increases the cost of the processing operation and unnecessarily exposes the consumer to an excess of chemicals.

The inventor has discovered that spraying the antimicrobial preparation onto the face of each slice as it is created with approximately 80% coverage of the surface area is effective to yield full coverage when the slice is stacked and further processed as discussed herein. As each sprayed slice falls onto the previous slice, the back end of that slice is coated with the antimicrobial preparation as well, via contact with the next slice to fall on the stack. In an embodiment of the invention, each shot is dispensed only when the slicer is cutting meat and is applied to the face of the meat in between each revolution or oscillation of the slicing blade. In a further embodiment of the invention, the system accommodates blade speeds from 0-1500 RPM, i.e., up to 1500 slices per minute.

Once each slice in a stack of food product, e.g., deli meat, (typically 6-12 slices) has been sprayed, the same system will also dispense another small shot of antimicrobial preparation into a vacuum pouch, e.g., created by a rollstock machine. The rollstock packaging machine or other system for providing the vacuum packaging is located at the outlet end of the slicing system in an embodiment of the invention. The additional amount of antimicrobial preparation acts to treat the bottom, top, and sides of the packaged stack. Although the antimicrobial preparation is added to the package before insertion of the stack, as a vacuum is drawn to seal the package, the antimicrobial is dispersed across the surface area of the stack.

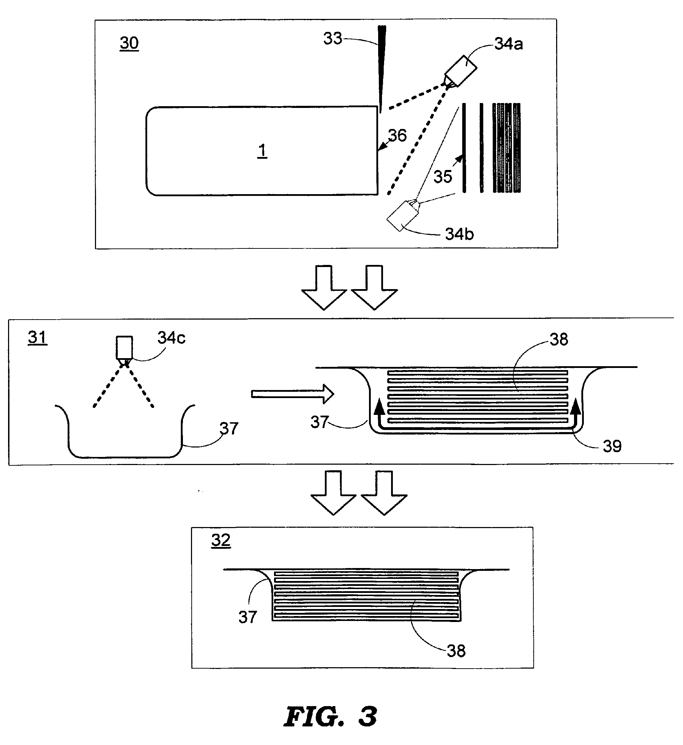

In keeping with the preceding discussion, FIG. 3 shows a schematic diagram of a meat slicing system according to an embodiment of the present invention. The system is divided into a slicing stage 30, a packaging phase 31, and a finishing stage 32. Although these stages 30, 31, 32 may operate continuously and simultaneously, they can be considered to operate sequentially with respect to a given slice stack. Moreover, it will be appreciated that although only one processing chain is shown, it is contemplated in an embodiment of the invention that multiple processing chains like that shown in FIG. 3 will be run simultaneously.

At the slicing stage 31, a slicer blade 33 produces sequential slices of a food product from a log 1 as discussed above. However, in addition, a first spray nozzle 34a located adjacent the log 1 (but out of the path of travel for the slices) sprays a shot of antimicrobial preparation on the exposed face 36 of the log 1. This face 36 will become the rear side of the next slice to be removed from the log 1. In an optional embodiment of the invention, as each slice is removed, a second optional spray nozzle 34b may apply a second shot of antimicrobial preparation to the reverse side 35 of the slice.

In an embodiment of the invention, the first and second spray nozzles 34a, 34b are triggered intermittently by an optical or other sensor or output (not shown) that provides a signal indicating the position of the blade 33. In another embodiment, an encoder signal from the slicer is used to establish position of the blade, and thus is used to set the window and timing of the shot. Thus, for example, when the blade 33 is in a first position A so as to expose the face 36 of the log 1, the first spray nozzle 34a is activated for a predetermined burst period. As each slice falls away from the log 1 (e.g., when the blade 33 is in a second position *B* obscuring the face 36 of the log 1, the second spray nozzle 34b is activated for a second predetermined burst period. When the second burst is applied, the removed slice may be either on the stack in progress or in transit to the stack. Except for the first and last slices in the stack, when each slice contacts the stack in progress, the antimicrobial preparation on the surfaces 36, 35 of the slice is spread by contact with the preceding and following slices respectively.

As each stack is completed, it is moved to a packaging area (which may be adjacent the slicing area) where a packaging operation 31 is executed. As part of the packaging operation 31, a third spray nozzle 34c provides a shot of antimicrobial preparation into a vacuum rollstock pouch 37. The timing of this shot from the third spray nozzle 34c is not dependent upon the timing of the blade 33 or the other spray nozzles 34a, 34b. Rather, it is just necessary to execute the shot from the third spray nozzle 34c sometime prior to packaging the stack. The signal for triggering this third shot may be derived from the rollstock machine itself rather than a push button or external sensor in an embodiment of the invention.

Once the stack 38 is in the packaging area and the shot from the third spray nozzle 34c has been executed, the stack 38 is inserted into the vacuum pouch 37 as shown in stage 31. Subsequently, a vacuum is applied to the interior of the pouch 37. The applied vacuum causes the antimicrobial preparation 39 deposited by the shot from the third spray nozzle 34c to be drawn across the surface of the stack 37, coating any external surfaces that may not have been reached by the shots from the first spray nozzle 34a and the optional second spray nozzle 34b. Once the appropriate vacuum has been drawn, the vacuum pouch 37 is sealed at the sealing stage 32.

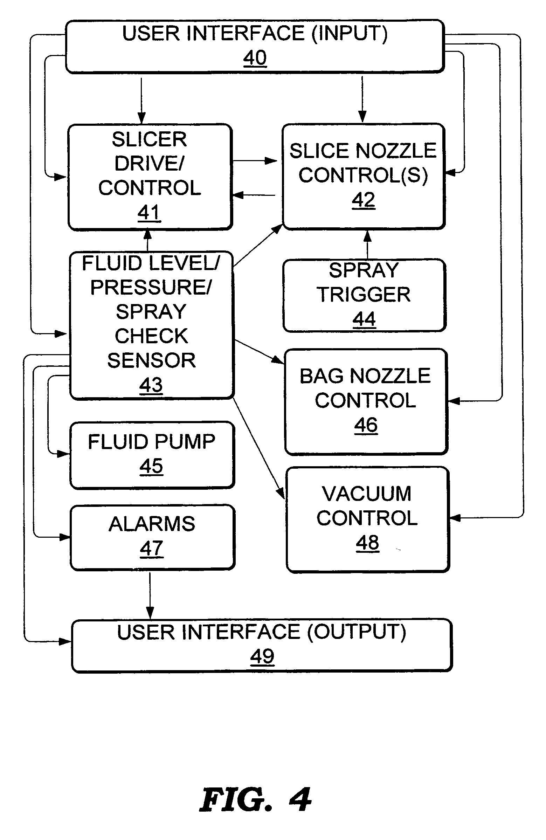

Having discussed the overall structure and operation of the system, the control and communication architecture of the system will be discussed in greater detail with reference to FIG. 4. As will be appreciated, FIG. 4 is a system schematic corresponding to a system within an embodiment of the present invention, including elements for user input and output, spray control, packaging control, and alarm control.

The system illustrated in FIG. 4 includes an input user interface 40 for receiving input from a system operator, e.g., to start and stop processes, to set alarm levels and spray shot durations, etc. An output user interface 49 is included in the system to provide information to the user, e.g., feedback as values are entered in the input interface 40, alarms during operations, and other signaling and informational output.

In order to execute the user-programmed operation, the system also includes a number of other components including a slicer drive/control 41 for controlling the operation of the slicing blade. This control module may be external to the slicer machine itself or may be embedded in the slicer. The control 41 may be of any suitable design, but in an embodiment of the invention it is an electric drive control of an AC or DC configuration, which controls the speed of the slicer blade, and thus controls the frequency at which new slices are processed. Suitable controls include but are not limited to voltage level controls, current level controls, stepper controls, pulse width modulation controls, and so on, and may, but need not, employ feedback in a speed control loop.

For controlling the first spray nozzle 34a and the optional second spray nozzle 34b, slice nozzle controls 42 arc included in the system. If multiple processing chains are run simultaneously, this module may control all of the slice nozzles needed. These controls 42 activate the spray nozzles 34a, 34b based on a trigger signal received from a spray trigger 44. The spray trigger 44 may be of any suitable type, but in an embodiment of the invention the spray trigger 44 is a beam interruption trigger linked to a hole or other space or gap associated with the slicer blade 3, 33 or the drive 41. In a preferred embodiment of the invention, the spray trigger comprises an encoder signal received from the slicer, defining the slicer blade position.

In order to provide the shot of antimicrobial preparation to the vacuum pouch 37 prior to vacuum and sealing, a rollstock pouch nozzle control 46 is provided. As noted above, the timing of this shot is not critical but it is desirable that the shot occur prior to insertion of the stack 38 into the pouch 37.

The antimicrobial preparation is supplied to the various nozzles in pressurized liquid form. A fluid pump 45 pressurizes the fluid from a tank to promote the flow and spray of the fluid. Thus, in order to ensure that the proper amount of preparation is applied, the timing and pressure of the fluid spray must be monitored and the availability of the fluid in the tank must be maintained. To this end, the system includes a tank fluid level sensor, pump fluid pressure sensor, and spray check sensor, represented collectively in FIG. 4 as the fluid level/pressure/spray check sensor 43 module.

The fluid level sensor may be a continuous sensor within the tank (not shown) or a float type sensor. If the fluid level drops below the low float level in the tank for an extended period of time, an audible and/or visible alarm may also be triggered by the system. The fluid pressure sensor may be a P/I transducer or other suitable device for measuring pressure. In an embodiment of the invention, the pressure of the fluid is maintained within a predetermined variance, e.g., 30 PSI. If the pressure exceeds or drops below this tolerance, an alarm may sound, the stack light may activate, and another alarm notification may appear on the touch screen (e.g., on user interface 40 and/or user interface 49). In an embodiment of the invention, the tank is auto-filled on a continuous basis rather than being periodically filled. In a further embodiment of the invention, the tank itself may be pressurized and a pressure relief valve may be used to maintain fluid pressure at a predetermined level during filling operations.

In order to provide the vacuum required to appropriately evacuate the vacuum pouch 37 and uniformly spread the final shot of antimicrobial preparation, a vacuum control 48 manages the application of vacuum pressure to the pouch 37. In an embodiment of the invention, the rollstock machine independently manages this operation and is not tied into the slicer or spray system. In an embodiment of the invention, the vacuum control 48 includes driving circuitry and a solenoid valve. It will be appreciated that other arrangements are possible as well.

To check the pulse duration of the various antimicrobial applications, one or more spray check sensors may be situated near the nozzles 34a, 34b (optional), 34c. The spray check sensors may be optical or otherwise, and provide a signal indicating the duration of the respective shots. If a shot duration varies from its nominal value by more than a predetermined margin for more than a predetermined period of time or number of occurrences, an alarm condition is considered to have occurred, and it is deemed that the necessary amount of preparation was not applied. For example, a sequence of three consecutive non-conforming shots is sufficient to trigger an alarm in an embodiment of the invention.

In a particular embodiment of the invention, deviations above the nominal time may be ignored. In a further related embodiment of the invention, if the registered spray time from the sensor is equal to or greater than 80% of the calculated shot size, this is considered to be a good shot. If the shot size is less than 80% for the required time or number of occurrences, an alarm will be displayed on the stack light and on the touch screen of the unit to identify which nozzle had the alarm.

To provide the necessary alarms to the operator, an alarm module 47 processes information received from the various sensors and provides and audible and/or visual alarm to the operator if an alarm condition is indicated by the sensor values.

In summary, the digital inputs received by the system to operate and to verify correct operation are a spray check sensor signal, a trigger signal (e.g., from a physical push button or thru beam photo eye), a fluid level switch signal (low level tank). In an embodiment of the invention, a level switch is also provided to monitor a low level in a concentrate tank. The digital outputs provided by the system during operation include nozzle on/off signals and alarm signals.

The analog inputs used by the system include a pressure transmitter signal and a continuous level sensor signal where such a sensor is used. The analog outputs provided by the system during operation include primarily the air pressure output control signal where used.

As noted above, it is important to apply the correct amount of antimicrobial preparation to the product to economically and effectively control bacterial outgrowth. In an embodiment, the volume sprayed for each slice ranges from about .3 milliliter to about 1 milliliter. In an embodiment of the invention, the operator inputs a shot size in milliliters based on product size and surface area. In an exemplary implementation, each nozzle is a solid stream type nozzle with approximately a 3.124 mm (0.123") size orifice. In another embodiment of the invention, the shot sizes vary from 5 milliliters to 40 milliliters. In order to allow high production speed, all shot sizes may be required to be dispensed within a short time period, e.g., .5 seconds in an embodiment of the invention.

By way of example, at an operating pressure of 125 PSI, a 2.388 mm (0.094") orifice will dispense approximately 22.71 l/min (0.6 GPM (gallons per minute)). The program takes into account the programmed shot size and converts it into a flow rate by using the relationship of P₁/P₂ = Q₁^{1/2}/Q₂^{1/2}. Using this formula, a shot time is calculated to deliver the programmed shot size volume.

When an autofilling function and pressurized tank are desired, the program operates by adjusting a programmable pressure set point for the tank. For a whole muscle based system, for example, this pressure is set at 30 PSI. In other applications, the tank is at ambient pressure, and a pump subsequently pressurizes fluid at it exits the tank, e.g., to 120 PSI.

When the tank level falls below a programmed threshold (typically above 5%), more fluid is allowed into the tank. In an embodiment, this action is executed via a mixing pump having an outlet valve connecting into the tank. The mixing pump intakes water and concentrate, and produces the appropriately diluted antimicrobial preparation. The formula for the antimicrobial preparation may vary, e.g., depending upon whether the purpose of the system is to only kill pathogenic bacteria or also to prolong the shelf life of the packaged product.

This auto fill function will allow the tank to start refilling while the system is still in operation. As the pressure starts building in the tank during the refill cycle, the excess air pressure in the tank is bled off by the I/P to ensure that the pressure stays constant during the refill cycle. In a further embodiment of the invention, the system includes a concentrate tank with a low level float type level sensor. If the fluid level drops below the low mark, an audible and visual alarm is activated in the panel.

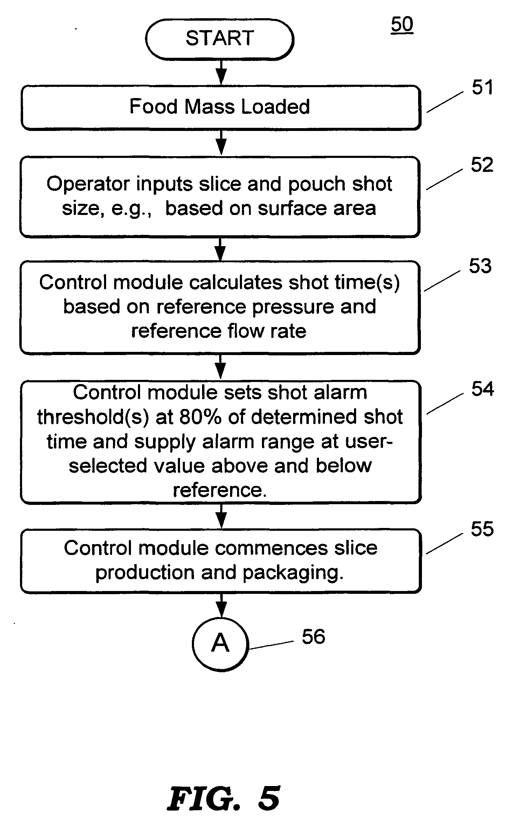

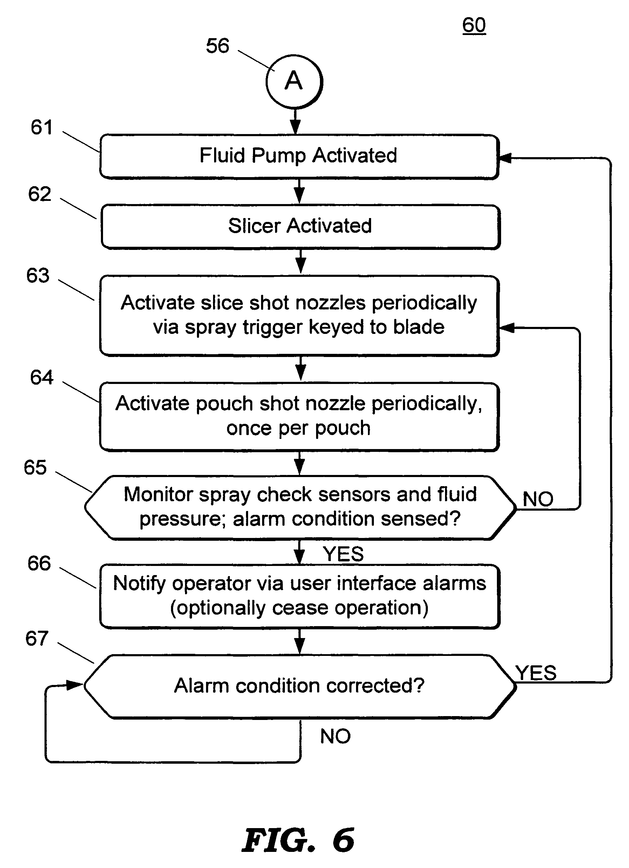

The process steps discussed herein that are not executed by the operator are executed via computer control, e.g., via a computing device reading computer-executable instructions from a computer-readable medium such as a disk, RAM, ROM, thumb drive, etc. In the description of FIGS. 5 and 6, this will not be repeated at length, but those of skill in the art will be aware that the non-operator executed steps are executed automatically under computer control as described above.

FIG. 5 is a flow chart illustrating a process 50 for initializing a meat processing system in an embodiment of the present invention. At stage 51, a food mass, e.g., a meat log 1, is loaded in the machine. This step will typically be operator-executed, but may also be automated if desired. At stage 52, the operator inputs slice and rollstock/ pouch shot size, e.g., based on surface area of the loaded food product. The control module calculates shot times based on reference pressure and reference flow rates at stage 53 in the manner discussed above.

At stage 54, the control module sets shot alarm thresholds at 80% of the determined shot time (or other desired cut-off) and supplies alarm ranges at user-selected values above and below this reference level. At stage 55, the control module commences slice production and packaging. From this stage, the process 50 moves to waypoint A (56).

The process 60, illustrated in the flow chart of FIG. 6, begins from waypoint A (56). At stage 61 of the process 60, the fluid pump activated, and the slicer is activated subsequently at stage 62. The slice shot nozzles are triggered periodically via the spray trigger from the slicing machine at stage 63, and the rollstock/pouch shot nozzle is activated periodically, once per pouch, at stage 64.

As the process progresses, the controller monitors the spray check sensors and fluid pressure and level signals. If an alarm condition is sensed at stage 65, the system may execute a user-selected remedial action. In an embodiment, a manually selectable option allows the process to shut down or to continue operation if alarm condition arises. In either case, the system preferably notifies the operator via user interface alarms at stage 66. Otherwise, the system returns to stage 63 to continue processing.

In the event that an alarm condition was indicated at stage 66, the system awaits correction of the condition at stage 67. If the alarm condition is corrected, the system flows to stage 61 to reinitiate processing. Otherwise, the system loops at stage 67 and continues to await correction of the error condition.

It will be appreciated that the structures and process presented herein enable and new and more efficient way to process sliced food products.

The use of the terms "a" and "an" and "the" and similar referents in the context of describing the invention (especially in the context of the following claims) are to be construed to cover both the singular and the plural, unless otherwise indicated herein or clearly contradicted by context The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventor for carrying out the invention.

## Claims

1. A system for preparing packages of sliced food product from a piece (1) of said food product while minimizing bacterial contamination and outgrowth within the packaged food product, the system having a powered slicer having a blade (3; 33) and a support (2) for holding the piece of food product (1) and moving it gradually into the blade (3; 33) of the powered slicer, the blade acting to sequentially separate slices (4) of the food product from the piece of food product (1) to create a stack (6; 38) of food product slices, thereby exposing a first face (35, 36) of the piece of food product (1) when each said slice (4) is removed, the system being further **characterized by**:
a fluid source for providing a liquid antimicrobial preparation, a first spray nozzle (34a, 34b) for depositing a first predetermined amount of the antimicrobial preparation onto the first face (35, 36) each time a slice (4) is removed from the piece of food product (1), a vacuum pouch system for providing a vacuum pouch (37) to receive each stack (6; 38) of food product slices, and a second spray nozzle (34c) for depositing a second predetermined amount of the antimicrobial preparation into each vacuum pouch (37) prior to reception of a stack (6; 38) within the pouch (37).

2. The system for preparing packages of sliced food product according to claim 1, wherein the system is further **characterized by** a vacuum pump for applying a vacuum to each vacuum pouch (37) after reception of a stack (6; 38) within the pouch such that the second predetermined amount of the antimicrobial preparation is substantially evenly spread over an external surface of the stack (6; 38).

3. The system for preparing packages of sliced food product according to either of claims 1 and 2, further **characterized in that** the first spray nozzle (34a, 34b) is intermittently activated based on a position of the blade (3; 33) of the powered slicer.

4. The system for preparing packages of sliced food product according to any of claims 1-3, wherein the second spray nozzle (34c) is intermittently activated.

5. The system for preparing packages of sliced food product according to any of claims 1-4, further **characterized in that** the fluid source for providing a liquid antimicrobial preparation further comprises a first tank providing liquid antimicrobial preparation to the first spray nozzle (34a, 34b) and a second tank providing liquid antimicrobial preparation to the second spray nozzle (34c).

6. The system for preparing packages of sliced food product according to claim 5, wherein the first tank is at a first pressure and the second tank is at a second pressure different from the first pressure.

7. The system for preparing packages of sliced food product according to claim 6, further comprising a liquid pump (45) between the first tank and the first spray nozzle (34a, 34b) for providing a pressurized flow of liquid antimicrobial preparation to the first spray nozzle (34a, 34b).

8. The system for preparing packages of sliced food product according to any of claims 1-7, further including a spray check sensor (43) associated with the first spray nozzle (34a, 34b) to determine the duration of spray shots emanating from the first spray nozzle (34a, 34b).

9. The system for preparing packages of sliced food product according to claim 8, further comprising an alarm module (47) for notifying an operator of the system if the spray check sensor (43) associated with the first spray nozzle (34a, 34b) detects a predetermined alarm condition.

10. The system for preparing packages of sliced food product according to claim 9, wherein the predetermined alarm condition identifies a number of consecutive spray occurrences delivering less than a predetermined amount of the antimicrobial preparation.

## Patentansprüche

1. System zur Herrichtung von Paketen eines in Scheiben geschnittenen Nahrungsmittelproduktes aus einem Stück (1) des Nahrungsmittelproduktes unter Minimierung bakterieller Verunreinigung und bakteriellen Auswuchses innerhalb des verpackten Nahrungsmittelproduktes, wobei das System eine angetriebene Schneidvorrichtung mit einem Messer (3; 33) und eine Stütze (2), um das Stück des Nahrungsmittelproduktes (1) zu halten und dieses sukzessive in das Messer (3; 33) der angetriebenen Schneidvorrichtung zu bewegen, wobei das Messer arbeitet, um sequentiell Scheiben (4) des Nahrungsmittelproduktes aus dem Stück des Nahrungsmittelproduktes (1) abzutrennen, um einen Stapel (6; 38) der Nahrungsmittelproduktscheiben zu erzeugen, wodurch eine erste Stirnfläche (35, 36) des Stückes des Nahrungsmittelproduktes (1) freigelegt wird, wenn jede Scheibe (4) entfernt wird, wobei das System ferner **gekennzeichnet ist durch**:
eine Fluidquelle zur Bereitstellung eines flüssigen antimikrobiellen Präparates, eine erste Sprühdüse (34a, 34b) zur Aufbringung einer ersten vorbestimmten Menge des antimikrobiellen Präparates auf die erste Stirnfläche (35, 36) jedes Mal, wenn eine Scheibe (4) aus dem Stück des Nahrungsmittelproduktes (1) entfernt wird, ein Vakuumbeutelsystem zur Bereitstellung eines Vakuumbeutels (37), um jeden Stapel (6; 38) der Nahrungsmittelproduktscheiben aufzunehmen, und eine zweite Sprühdüse (34c) zur Einbringung einer zweiten vorbestimmten Menge des antimikrobiellen Präparats in jeden Vakuumbeutel (37) vor der Aufnahme eines Stapels (6; 38) im Inneren des Beutels (37).

2. System zur Herrichtung von Paketen eines in Scheiben geschnittenen Nahrungsmittelproduktes nach Anspruch 1, wobei das System ferner **gekennzeichnet ist durch** eine Vakuumpumpe zum Anlegen von Vakuum an jeden Vakuumbeutel (37) nach der Aufnahme eines Stapels (6; 38) im Inneren des Beutels, so dass die zweite vorbestimmte Menge des antimikrobiellen Präparats im Wesentlichen gleichmäßig über eine äußere Oberfläche des Stapels (6; 38) verteilt wird.

3. System zur Herrichtung von Paketen eines in Scheiben geschnittenen Nahrungsmittelproduktes nach einem der Ansprüche 1 und 2, das ferner **dadurch gekennzeichnet ist, dass** die erste Sprühdüse (34a, 34b) basierend auf einer Position des Messers (3; 33) der angetriebenen Schneidvorrichtung intermittierend betätigt wird.

4. System zur Herrichtung von Paketen eines in Scheiben geschnittenen Nahrungsmittelproduktes nach einem beliebigen der Ansprüche 1-3, wobei die zweite Sprühdüse (34c) intermittierend aktiviert wird.

5. System zur Herrichtung von Paketen eines in Scheiben geschnittenen Nahrungsmittelproduktes nach einem beliebigen der Ansprüche 1-4, das ferner **dadurch gekennzeichnet ist, dass** die Fluidquelle zur Bereitstellung eines flüssigen antimikrobiellen Präparats ferner einen ersten Behälter, der ein flüssiges antimikrobielles Präparat für die erste Sprühdüse (34a, 34b) bereitstellt, und einen zweiten Behälter aufweist, der ein flüssiges antimikrobielles Präparat für die zweite Sprühdüse (34c) bereitstellt.

6. System zur Herrichtung von Paketen eines in Scheiben geschnittenen Nahrungsmittelproduktes nach Anspruch 5, wobei der erste Behälter unter einem ersten Druck steht und der zweite Behälter unter einem zweiten Druck steht, der sich von dem ersten Druck unterscheidet.

7. System zur Herrichtung von Paketen eines in Scheiben geschnittenen Nahrungsmittelproduktes nach Anspruch 6, das ferner eine Flüssigkeitspumpe (45) zwischen dem ersten Behälter und der ersten Sprühdüse (34a, 34b) zur Zuführung einer unter druckbeaufschlagten Strömung eines flüssigen antimikrobiellen Präparats zu der ersten Sprühdüse (34a, 34b) aufweist.

8. System zur Herrichtung von Paketen eines in Scheiben geschnittenen Nahrungsmittelproduktes nach einem beliebigen der Ansprüche 1-7, das ferner einen Sprühüberwachungssensor (43) enthält, der der ersten Sprühdüse (34a, 34b) zugeordnet ist, um die Dauer der aus der ersten Sprühdüse (34a, 34b) ausströmenden Sprühstöße zu bestimmen.

9. System zur Herrichtung von Paketen eines in Scheiben geschnittenen Nahrungsmittelproduktes nach Anspruch 8, das ferner ein Alarmmodul (47) zur Benachrichtigung eines Bedieners des Systems in dem Fall, dass der Sprühüberwachungssensor (43), der der ersten Sprühdüse (34a, 34b) zugeordnet ist, einen vorbestimmten Alarmzustand detektiert, aufweist.

10. System zur Herrichtung von Paketen eines in Scheiben geschnittenen Nahrungsmittelproduktes nach Anspruch 9, wobei der vorbestimmte Alarmzustand eine Anzahl aufeinanderfolgender Sprühereignisse, die weniger als eine vorbestimmte Menge des antimikrobiellen Präparats liefern, identifiziert.

## Revendications

1. Système de préparation d'emballages de produit alimentaire en tranches à partir d'une pièce (1) dudit produit alimentaire tout en minimisant la contamination bactérienne et sa croissance dans le produit alimentaire emballé, le système comportant un dispositif de coupe en tranches alimenté en énergie comportant une lame (3; 33) et un support (2) pour maintenir la pièce de produit alimentaire (1) et la déplacer progressivement dans la lame (3; 33) du dispositif de coupe en tranches alimenté en énergie, la lame agissant pour séparer séquentiellement des tranches (4) du produit alimentaire de la pièce de produit alimentaire (1) pour créer une pile (6; 38) de tranches de produit alimentaire, exposant ainsi une première face (35, 36) de la pièce de produit alimentaire (1) quand ladite tranche (4) est enlevée, le système étant en outre **caractérisé par** :
une source de fluide pour fournir une préparation antimicrobienne liquide, une première buse de pulvérisation (34a, 34b) pour déposer une première quantité prédéterminée de la préparation antimicrobienne sur la première face (35, 36) chaque fois qu'une tranche (4) est enlevée de la pièce de produit alimentaire (1), un système de sac sous vide pour fournir un sac sous vide (37) pour recevoir chaque pile (6; 38) de tranches de produit alimentaire, et une seconde buse de pulvérisation (34c) pour déposer une seconde quantité prédéterminée de la préparation antimicrobienne dans chaque sac sous vide (37) avant la réception d'une pile (6; 38) dans le sac (37).

2. Système de préparation d'emballages de produit alimentaire en tranches selon la revendication 1, dans lequel le système est **caractérisé en outre par** une pompe à vide pour appliquer un vide à chaque sac sous vide (37) après réception d'une pile (6; 38) dans le sac de telle manière que la seconde quantité prédéterminée de la préparation antimicrobienne est sensiblement répandue de manière sensiblement égale sur une surface extérieure de la pile (6; 38).

3. Système de préparation d'emballages de produit alimentaire en tranches selon l'une quelconque des revendications 1 et 2, **caractérisé en outre en ce que** la première buse de pulvérisation (34a, 34b) est activée de manière intermittente en fonction d'une position de la lame (3; 33) du dispositif de coupe en tranches alimenté en énergie

4. Système de préparation d'emballages de produit alimentaire en tranches selon l'une quelconque des revendications 1-3, dans lequel la seconde buse de pulvérisation (34c) est activée de manière intermittente.

5. Système de préparation d'emballages de produit alimentaire en tranches selon l'une quelconque des revendications 1-4, **caractérisé en outre en ce que** la source de fluide pour fournir une préparation antimicrobienne liquide comprend en outre un premier réservoir fournissant une préparation antimicrobienne liquide à la première buse de pulvérisation (34a, 34b) et un second réservoir fournissant une préparation antimicrobienne liquide à la seconde buse de pulvérisation (34c).

6. Système de préparation d'emballages de produit alimentaire en tranches selon la revendication 5, dans lequel le premier réservoir est à une première pression et le second réservoir est à une seconde pression différente de la première pression.

7. Système de préparation d'emballages de produit alimentaire en tranches selon la revendication 6, comprenant en outre une pompe à liquide (45) entre le premier réservoir et la première buse de pulvérisation (34a, 34b) pour fournir un flux sous pression de préparation antimicrobienne liquide à la première buse de pulvérisation (34a, 34b).

8. Système de préparation d'emballages de produit alimentaire en tranches selon l'une quelconque des revendications 1-7, incluant en outre un capteur de clapet de pulvérisateur (43) associé à la première buse de pulvérisation (34a, 34b) pour déterminer la durée de jets de pulvérisation émanant de la première buse de pulvérisation (34a, 34b).

9. Système de préparation d'emballages de produit alimentaire en tranches selon la revendication 8, comprenant en outre un module d'alarme (47) pour notifier à un opérateur du système si le capteur de clapet de pulvérisateur (43) associé à la première buse de pulvérisation (34a, 34b) détecte une condition d'alarme prédéterminée.

10. Système de préparation d'emballages de produit alimentaire en tranches selon la revendication 9, dans lequel la condition d'alarme prédéterminée identifie un nombre d'occurrence de pulvérisation consécutives inférieur à une quantité prédéterminée de la préparation antimicrobienne.
